# EUROPEAN PATENT SPECIFICATION

(11) **EP 0 462 721 B1**
(45) Date of publication and mention of the grant of the patent: **22.08.2001**
(21) Application number: 91305047.2
(22) Date of filing: 04.06.1991
(51) Int. Cl.: C12N 15/32, C12P 21/02, C12N 1/20, A01N 63/00, A01H 5/00

(54) **Novel Bacillus thuringiensis microbes active against nematodes, and genes encoding novel nematode-active toxins cloned from Bacillus thuringiensis isolates**
Gegen Nematodes aktive Bacillus thuringiensis-Mikroben, und Gene kodierend für Nematode-aktive Toxine kloniert von Bacillus thuringiensis-Stämmen
Microbes de Bacillus thuringiensis actifs contre des nématodes, et gènes, codant pour des toxines actives contre des nématodes, clonés de souches de Bacillus thuringiensis

(30) Priority: 11.06.1990 US 535810; 24.07.1990 US 557246; 27.07.1990 US 558738; 10.08.1990 US 565544; 14.03.1991 US 669126; 27.03.1991 US 675772; 03.05.1991 US 693018
(43) Date of publication of application: 27.12.1991
(60) Divisional application: 01102789.3
(73) Proprietor: MYCOGEN CORPORATION, San Diego, California 92121 (US)
(72) Inventor: Narva, Kenneth E., San Diego, California 92130 (US); Payne, Jewel M., San Diego, California 92126 (US); Schwab, George E., La Jolla, California 93037 (US); Hickle, Leslie Ann, San Diego, California 92115 (US); Galasan, Theresa, San Diego, California 92130 (US); Sick, August J., Oceanside, California 92056 (US)
(74) Representative: Perry, Robert Edward

(56) References cited:
- EP-A- 0 303 426
- EP-A- 0 352 052
- JOURNAL OF THE KANSAS ENTOMOLOGICAL SOCIETY, vol. 50, no. 3, July 1977, KANSAS, US, pages 394-398; C.M. IGNOFFO ET AL.: "Deleterious effects of the thermostable toxin of Bacillus thuringiensis on species of soil-inhabiting Myceliophagus, and plant-parasitic nematodes"

## Description

### Background of the Invention

Regular use of chemicals to control unwanted organisms can select for drug resistant strains. This has occurred in many species of economically important insects and has also occurred in nematodes of sheep, goats, and horses. The development of drug resistance necessitates a continuing search for new control agents having different modes of action.

In recent times, the accepted methodology for control of nematodes has centered around the drug benzimidazole and its congeners. The use of these drugs on a wide scale has led to many instances of resistance among nematode populations (Prichard, R.K et al. [1980] "The problem of anthelmintic resistance in nematodes," Austr. Vet. J. 56:239-251; Coles, G.C. [1986] "Anthelmintic resistance in sheep," In Veterinary Clinics of North America: Food Animal Practice, Vol 2:423-432 [Herd, R.P., eds.] W.B. Saunders, New York). There are more than 100,000 described species of nematodes.

The bacterium Bacillus thuringiensis (B.t.) produces a δ-endotoxin polypeptide that has been shown to have activity against a rapidly growing number of insect species. The earlier observations of toxicity only against lepidopteran insects have been expanded with descriptions of B.t. isolates with toxicity to dipteran and coleopteran insects. These toxins are deposited as crystalline inclusions within the organism. Many strains of B.t. produce crystalline inclusions with no demonstrated toxicity to any insect tested.

A small number of research articles have been published about the effects of delta endotoxins from B. thuringiensis species on the viability of nematode eggs. Bottjer, Bone and Gill (Experimental Parasitology 60:239-244, 1985) have reported that B.t. kurstaki and B.t. israelensis were toxic in vitro to eggs of the nematode Trichostrongylus colubriformis. In addition, 28 other B.t. strains were tested with widely variable toxicities. The most potent had LD₅₀ values in the nanogram range. Ignoffo and Dropkin (Ignoffo, C.M. and Dropkin, V.H. [1977] J. Kans. Entomol. Soc. 50:394-398) have reported that the thermostable toxin from Bacillus thuringiensis (beta exotoxin) was active against a free-living nematode, Panagrellus redivivus (Goodey); a plant-parasitic nematode, Meloidogyne incognita (Chitwood); and a fungus-feeding nematode, Aphelenchus avena (Bastien). Beta exotoxin is a generalized cytotoxic agent with little or no specificity. Also, H. Ciordia and W.E. Bizzell (Jour. of Parasitology 47:41 [abstract] 1961) gave a preliminary report on the effects of B. thuringiensis on some cattle nematodes.

Flukes belong to subclass Digenea, class Trematoda in the phylum Platyhelminthes. These digeneans all have an intermediate host and are found exclusively in vertebrates, including man. Families which have members of considerable veterinary importance are Fasciolidae, Dicrocoeliidae, Paramphistomatidae, and Schistosomatidae. The adult trematode flukes occur primarily in the bile ducts, alimentary tract, and vascular system. Depending on the predilection site, the eggs pass out of the final host, usually in faeces or urine, and the larval stages develop in a molluscan intermediate host.

Several clinical syndromes may be associated with liver fluke (Fasciola sp.) infection, depending on the numbers and stage of development of the parasite and on the presence or absence of certain bacteria (Clostridium novyi). Acute fluke disease occurs during invasion of the liver by recently ingested metacercariae. Sheep can die very quickly due to focal liver necrosis and extensive subcutaneous hemorrhage.

Anthelmintic medication in the U.S. currently consists primarily of albendazole, which is available in only a few states where Fasciola hepatica poses a serious problem. Special dispensation is required to treat sheep with albendazole elsewhere in the U.S. Other effective flukicides―diamphenethide, nitroxynil, oxyclozanide, rafoxanide, and triclabendazole- are not available in the U.S.

There is a need to have more effective means to control the many nematodes and flukes that cause considerable damage to susceptible hosts. Advantageously, such means would employ biological agents.

### Summary of the Invention

The subject invention concerns the novel *Bacillus thuringiensis* strain PS80JJ1, that produces a biologicallyactive toxin. This toxin has been shown to be active against nematodes. Specifically exemplified is activity against *Panagrellus redivivus* (also called the beer-mat nematode), a common free-living nematode that is relatively easy to maintain in the laboratory. It is often used as an indicator (model) of nematode activity. The toxins of the subject invention can also be used to control flukes, including the liver fluke *Fasciola hepatica.*

The *B.t.* isolate according to the subject invention can be grown, and the δ-endotoxin that is produced recovered by standard procedures and as further described herein. The recovered toxin, of the *B.t.* isolate itself, can be formulated using standard procedures associated with the use of nematicidal or flukicidal products.

A gene coding for the toxin can also be obtained from *B.t.* PS80JJ1.

A subculture of the novel *Bacillus thuringiensis* isolate was deposited on 17.07.90 in the permanent collection of the Northern Research Laboratory, U.S. Department of Agriculture, Peoria, Illinois, USA. The accession number is NRRL B-18679.

### Description of the Invention

The novel toxin shows activity against tested nematodes. The group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes wide-spread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris, Caenorhabditis and Parascaris. Certain of these, such as Nematodirus, Cooperia, and Oesophagostomum, attack primarily the intestinal tract, while others, such as Dictyocaulus are found in the lungs. Still other parasites may be located in other tissues and organs of the body.

The toxin of the subject invention is useful as a nematocide for the control of soil nematodes and plant parasites selected from the genera Bursaphalenchus, Criconemella, Ditylenchus, Globodera, Helicotylenchus, Heterodera, Melodiogyne, Pratylenchus, Radolpholus, Rotelynchus, or Tylenchus.

The methods and compositions of the subject invention can also be used to control liver flukes, which can parasitize vertebrates. Specifically, the invention can be used to control flukes in humans, livestock, domestic pets, and other animals. As used herein, the term "livestock" can include, for example, sheep, cattle, pigs, and goats. The methods and compositions of the subject invention may be used to control immature and adult flukes. The methods of control include, but are not limited to, pasture treatment (for vertebrate and intermediate hosts); liposomes or other carriers for delivering the toxins to the liver or bile ducts; and treatment of free-living forms of the flukes in the gastrointestinal tracts of vertebrate hosts. The flukicidal B.t. toxin described herein may be used alone, or in rotation or combination with other flukicides such as, for example, albendazole.

As described herein, the B.t. isolate of the invention has activity against liver flukes. It is expected that these isolates would be active against other flukes as disclosed herein.

The B.t. toxin of the invention can be administered orally in a unit dosage form such as a capsule, bolus or tablet, or as a liquid drench when used as an anthelmintic in mammals and in the soil to control plant nematodes. The drench is normally a solution, suspension or dispersion of the active ingredient, usually in water, together with a suspending agent such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight, the capsules and boluses comprise the active ingredient admixed with a carrier vehicle such as starch, talc, nagnesium stearate, or dicalcium phosphate.

Where it is desired to administer the toxin in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the active agent, depending upon the factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or, optionally, fed separately. Alternatively, the toxin compounds may be administered to animals parenterally, for example, by intraruminal, intramuscular, intratracheal, or subcutaneous injection, in which event the active ingredient is dissolved or dispersed in a liquid carrier vehicle. For parenteral administration, the active material is suitably admixed with an acceptable vehicle, preferably of the vegetable oil variety, such as peanut oil, cotton seed oil and the like. Other parenteral vehicles, such as organic preparations using solketal, glycerol, formal and aqueous parenteral formulations, are also used. The active compound or compounds are dissolved or suspended in the parenteral formulation for administration; such formulations generally contain from 0.005 to 5% by weight of the active compound.

When the toxin is administered as a component of the feed of the animals, or dissolved or suspended in the drinking water, compositions are provided in which the active compound is intimately dispersed in an inert carrier or diluent. By inert carrier is meant one that will not react with the active agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like.

In addition to having nematicidal and flukicidal activity within the digestive tract of mammals, spores from the B.t. isolate will pass through the animals' digestive tract, germinate and multiply in the feces, and thereby provide additional control of larva which hatch and multiply therein.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The toxin concentration will vary widely depending upon the nature of the particular formulation, particularly whether it is a concentrate or to be used directly. The toxin will be present in at least 1% by weight and may be 100% by weight. The dry formulations will have from about 1-95% by weight of the toxin while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the nematodes or flukes, e.g., plants, soil or water, by spraying, dusting, sprinkling, or the like.

Following are examples which illustrate procedures, including the best mode, for practicing the invention. These examples should not be construed as limiting. All percentages are by weight and all solvent mixture proportions are by volume unless otherwise noted.

### Example 1 - Culturing B.t. Isolates

A subculture of the B.t. isolate can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salts Solution | 5.0 ml/l |
| CaCl₂ Solution | 5.0 ml/l |

| Salts Solution (100 ml) | |
|---|---|
| MgSO₄.7H₂O | 2.46 g |
| MnSO₄.H₂O | 0.04 g |
| ZnSO₄.7H₂O | 0.28 g |
| FeSO₄.7H₂O | 0.40 g |

| CaCl₂ Solution (100 ml) | |
|---|---|
| CaCl₂.2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The B.t. spores and crystals, obtained in the above fermentation, can be isolated by procedures well known in the art. A frequently-used procedure is to subject the harvested fermentation broth to separation techniques, e.g., centrifugation.

### Example 2 - Purification and Amino Acid Sequencing

The novel isolate can be cultured as described in Example 1 or by using other standard media and fermentation techniques well known in the art. The toxin protein inclusions can be harvested by standard sedimentation centrifugation. The recovered protein inclusions can be partially purified by sodium bromide (28-38%) isopycnic gradient centrifugation (Pfannenstiel, M.A., E.J. Ross, V.C. Kramer, and K.W. Nickerson [1984] FEMS Microbiol. Lett. 21:39). Thereafter the individual toxin proteins can be resolved by solubilizing the crystalline protein complex in an alkali buffer and fractionating the individual proteins by DEAE-sepharose CL-6B (Sigma Chem. Co., St. Louis, MO) chromatography by step-wise increments of increasing concentrations of an NaCl-containing buffer (Reichenberg, D., in Ion Exchangers in Organic and Biochemistry [C. Calmon and T.R.E. Kressman, eds.], Interscience, New York, 1957). Fractions containing protein toxic for the nematode Caenorhabditis elegans (CE), can be bound to PVDF membrane (Millipore, Bedford, MA) by western blotting techniques (Towbin, H., T. Staehelin, and K. Gordon [1979] Proc. Natl. Acad. Sci. USA 76:4350) and the N-terminal amino acids determined by the standard Edman reaction with an automated gas-phase sequenator (Hunkapiller, M.W., R.M. Hewick, W.L. Dreyer, and L.E. Hood [1983] Meth. Enzymol. 91:399).

### Example 3 - Activity against Panagrellus redivivus

Worms are collected in a tube and allowed to settle for about 15 minutes, and the water is decanted and replaced with fresh water three or four times until the water remains clear. 250 *µ* l rinsed nematodes (20-30 worms), and 100 *µ* l of a spore/crystal suspension of *B.t.* PS80JJ1 are added to 650 *µ*l water in each well of tray. Nematodes are counted and the numbers recorded. After four days, the live worms are counted. 99% mortality was calculated.

The approximate molecular masses of the alkali-soluble proteins in PS80JJ1 were 37, 47, 90 and 130 kDa.

## Claims

1. *Bacillus thuringiensis* strain PS80JJ1, NRRL B-18679.

2. Toxin active against nematodes, of approximately 130 kDa, which is obtainable from the strain of claim 1.

3. A process for controlling nematodes, which comprises contacting the nematodes with a toxin according to claim 2.

4. A method for controlling flukes, which comprises administering to a host harbouring a fluke, or directly to the fluke, or to the situs of the fluke, a toxin according to claim 2.

5. A nucleotide molecule encoding the toxin defined in claim 2.

## Patentansprüche

1. Bacillus thuringiensis-Stamm PS80JJ1, NRRL B-18679.

2. Toxin von etwa 130 kD mit Aktivität gegen Nematoden, welches aus dem Stamm nach Anspruch 1 erhältlich ist.

3. Verfahren zur Bekämpfung von Nematoden, welches das Kontaktieren der Nematoden mit einem Toxin nach Anspruch 2 umfaßt.

4. Verfahren zur Bekämpfung von Trematoden, welches umfaßt, daß einem Wirt, der einen Trematoden beherbergt, oder dem Trematoden direkt oder dem Aufenthaltsort des Trematoden ein Toxin nach Anspruch 2 verabreicht wird.

5. Nukleotidmolekül, kodierend für das Toxin nach Anspruch 2.

## Revendications

1. Souche de Bacillus thuringiensis PS80JJ1, NRRL B-18679.

2. Toxine active contre des nématodes, d'environ 130 kDa, qui peut être obtenue à partir de la souche de la revendication 1.

3. Procédé de contrôle des nématodes, comprenant la mise en contact des nématodes avec une toxine selon la revendication 2.

4. Méthode de contrôle des douves, comprenant l'administration d'une toxine selon la revendication 2 à un hôte abritant une douve ou bien directement à la douve ou bien à l'habitat de la douve.

5. Molécule nucléotidique codant pour la toxine définie dans la revendication 2.
